(19) Europäisches Patentamt
European Patent Office
Office européen des brevets

(11) **EP 4 596 576 A1**

(12) **EUROPEAN PATENT APPLICATION**
published in accordance with Art. 153(4) EPC

(43) Date of publication:
**06.08.2025 Bulletin 2025/32**

(21) Application number: **23872675.6**

(22) Date of filing: **06.04.2023**

(51) International Patent Classification (IPC):
*C07K 14/735* (2006.01)   *A61K 35/15* (2025.01)
*A61P 35/00* (2006.01)   *A61P 31/12* (2006.01)
*A61K 38/00* (2006.01)

(86) International application number:
**PCT/KR2023/004638**

(87) International publication number:
**WO 2024/071544 (04.04.2024 Gazette 2024/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.09.2022 KR 20220123421**

(71) Applicant: **Carots Co., Ltd.
Siheung-si, Gyeonggi-do 15011 (KR)**

(72) Inventors:
• **SHIN, Young Kee
Seoul 06279 (KR)**
• **LEE, Ji Hye
Hwaseong-si, Gyeonggi-do 18505 (KR)**
• **JO, Ji Won
Seoul 06978 (KR)**
• **KIM, Hong Sun
Incheon 21627 (KR)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **NOVEL RECOMBINANT FC RECEPTOR AND CELLS COMPRISING SAME**

(57)   The present invention relates to a recombinant Fc receptor and cells comprising same, and has cleavage resistance to ADAM17 through amino acid modification of an ADAM17 cleavage site in a CD16 polypeptide, and can improve binding affinity to an antibody Fc region by being substituted for all or a part of the extracellular binding domain of CD64.

[Figure 4b]

**Description**

TECHNICAL FIELD

The present invention relates to a novel recombinant Fc receptor and a cell including the same.

BACKGROUND ART

Immunotherapy, which is to treat a disease by activating or inhibiting the immune system, is a rapidly growing field with increasing clinical importance. In particular, the development of chimeric antigen receptor (CAR) T cells has brought about a breakthrough in adoptive cell transfer (ACT).

CAR is a receptor that is recombined to include one or more intracellular signaling domains and an antigen-binding domain for immune cell activation. Immune cells (CAR-X) that have been engineered to express CAR on their surfaces, such as cytotoxic T cells and natural killer cells (NK cells), are targeted by an antigen to which the antigen-binding domain binds, and are activated by binding to the antigen, thereby causing an immune response against cells expressing the antigen.

Over the past decade, a number of CARs targeting various cell surface antigens have been reported. In addition to the target antigen, compared to the first-generation CARs consisting of an antigen-binding domain, an intracellular signaling domain, and a transmembrane domain connecting them, various modifications have been attempted, such as the addition of a signaling domain derived from an immune costimulatory molecule and the combination of the entire domain features, in consideration of aspects such as cell proliferation, cytotoxicity, cytokine secretion, *in vivo* survival, immunocompatibility, and safety, and in particular, excellent effects have been exhibited in some blood cancers.

However, existing CARs have specificity only for certain antigens, and thus have various limitations. Cell therapy containing individual CARs involves several processes, including the discovery of target antigens specific to individual target diseased cells, the design/derivation of binding sites specific to the antigens, the design of CARs including them, and their introduction into cells. In addition, these processes require lengthy verification such as preclinical and clinical trials as well as procedures for deriving appropriate administration methods and compositions. Moreover, in the case of autologous CAR-Xs, which currently account for the majority, additional costs and time are required for the preparation process for each individual patient. In addition, cancers often show continuous mutations and heterogeneity not only between different patients but also between cells derived from the same patient, so the therapeutic effect of CAR-Xs with fixed target antigens on such cancers may be reduced. In addition, regarding existing CAR-X cells, there have been reports of risks such as cytokine storm (cytokine release syndrome) due to excessive activation of the cells, and toxicity to normal tissues having target antigens, including neurotoxicity (on-target, off-tumor effect). In addition, especially in solid tumors, the accessibility of immune cells is low, and even when immune cells successfully infiltrate the inside, an immunosuppressive microenvironment (tumor microenvironment) is often created, preventing the corresponding immune cells from being properly activated.

The present invention aims to provide a recombinant Fc receptor, which is a novel chimeric receptor that has universality and is capable of enhancing the activity of immune cells, overcoming the above-described limitations of existing CARs with fixed targets. The recombinant Fc receptor according to the present invention and the transformed mammalian cells including the same have improved the effects of immunotherapy and, since they do not have fixed target molecules, they can be applied to the treatment of various diseases. Furthermore, the recombinant Fc receptor is resistant to cleavage by ADAM17 that occurs after the activation of existing CD16, and thus can prevent the reduction of the immune activation effect of the receptor due to cleavage.

[Related Art Documents]

[Patent Document]

Korean Patent Publication No. 2021-0110827

DETAILED DESCRIPTION OF THE INVENTION

TECHNICAL PROBLEM

The present invention has been derived to solve the above-described problems, and an object of the invention is to provide a recombinant Fc receptor that may be used as a therapeutic agent for cancer or infectious diseases.

In addition, another object of the present invention is to provide a nucleic acid molecule encoding the recombinant Fc receptor.

In addition, still another object of the present invention is to provide an expression vector comprising the nucleic acid molecule.

In addition, yet another object of the present invention is to provide a host cell transformed with the expression vector.

In addition, yet another object of the present invention is to provide a pharmaceutical composition for treating cancer or a pharmaceutical composition for treating or preventing viral infection, including the host cell.

The technical problems to be solved by the present invention are not limited to the above-mentioned technical problems, and other technical problems that are not mentioned may be clearly understood by a person having ordinary skill in the technical field to which the present invention pertains, from the description below.

TECHNICAL SOLUTION

To solve the above-described technical, one embodiment of the present invention provides a recombinant Fc receptor including at least a part of a CD16 polypeptide including an ADAM17 cleavage site sequence corresponding to positions 196 to 198 of SEQ ID NO: 1, the recombinant Fc receptor including at least one of: i) at least one amino acid modification of substitution of serine at position 197 with proline and substitution of threonine at position 198 with serine; and ii) replacement of all or a part of an ectodomain of the CD16 polypeptide with an ectodomain of CD64.

In an embodiment of the present invention, the CD16 polypeptide may include an amino acid sequence of SEQ ID NO: 1, and the recombinant Fc receptor may include: at least one amino acid modification of substitution of the serine at amino acid position 197 with proline and substitution of the threonine at amino acid position 198 with serine; and substitution of the amino acid at position 176 of SEQ ID NO: 1 with valine.

In an embodiment of the present invention, the serine at amino acid position 197 may be substituted with proline, and all or a part of the ectodomain of the CD16 polypeptide may be replaced with an ectodomain of CD64.

In an embodiment of the present invention, the serine at amino acid position 197 may be substituted with proline, the threonine at amino acid position 198 may be substituted with serine, and all or a part of the ectodomain of the CD16 polypeptide may be replaced with the ectodomain of CD64.

In an embodiment of the present invention, all or a part of the ectodomain of the CD16 polypeptide may be replaced with the ectodomain of CD64.

In an embodiment of the present invention, the ectodomain of CD64 may include an amino acid sequence of SEQ ID NO: 2 or 3.

In an embodiment of the present invention, the recombinant Fc receptor may include any one amino acid sequence of SEQ ID NOs: 4 to 8.

Another embodiment of the present invention provides a nucleic acid molecule encoding the Fc receptor.

Still another embodiment of the present invention provides an expression vector including the nucleic acid molecule.

Yet another embodiment of the present invention provides a host cell transformed with the expression vector.

In an embodiment of the present invention, the host cell may be a mammalian immune cell, stem cell, or cell differentiated from a stem cell.

Yet another embodiment of the present invention provides a pharmaceutical composition for treating cancer, including the host cell.

Yet another embodiment of the present invention provides a pharmaceutical composition for treating viral infection, including the host cell.

The above-described means of solving the problems are merely exemplary and should not be construed as limiting the present invention. In addition to the above-described exemplary embodiments, additional embodiments may be present in the drawings and detailed description of the invention.

ADVANTAGEOUS EFFECTS

The recombinant Fc receptor according to an embodiment of the present invention can impart specificity for a desired antigen to a cell expressing the recombinant Fc receptor through binding to an antibody. According to this characteristic, the cell expressing the recombinant Fc receptor according to an embodiment of the present invention can be used for the treatment of various diseases depending on the type of antibody to be combined, and can also effectively respond to diseases such as cancer, which is subject to continuous mutations and heterogeneity between cells, by altering the type of antibody to be combined.

In addition, a recombinant Fc receptor according to one embodiment of the present invention can be resistant to cleavage by ADAM17 that occurs after binding of an antibody, and can have a higher affinity for the Fc domain of an antibody than CD16, thereby inducing a more effective effector response (e.g., antibody-dependent cellular cytotoxicity (ADCC), antibody-dependent cellular phagocytosis (ADCP), degranulation, cytokine secretion, etc.) in a cell expressing the recombinant Fc receptor.

In addition, a recombinant Fc receptor according to one embodiment of the present invention, when introduced into a cytotoxic effector cell (e.g., cytotoxic T lymphocyte (CTL)) that originally does not act antibody-dependently, enables the effector cell to activate an effector function by an antibody.

In addition, the cell according to one embodiment of the present invention is designed such that the Fc receptor included therein is expressed and regulated by the regulatory sequence of endogenous CD16. This enables reproduction of the expression pattern particularly in cells expressing CD16, such as natural killer cells (NK cells), and, when such cells are derived from stem cells, wasteful protein expression before differentiation can be prevented.

It should be understood that the effects of the present invention are not limited to the above-described effects but include all effects that may be inferred from the features of the invention described in the detailed description or claims of the present invention.

DESCRIPTION OF THE DRAWINGS

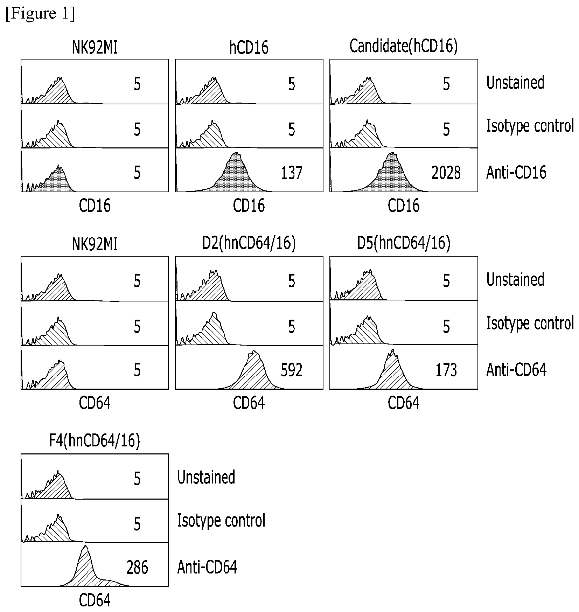

FIG. 1 shows the expression of CD16 or CD64 receptor, measured by flow cytometry, on the surface of NK92MI cells transfected with vectors including engineered receptors by electroporation, after sorting and antibiotic selection.

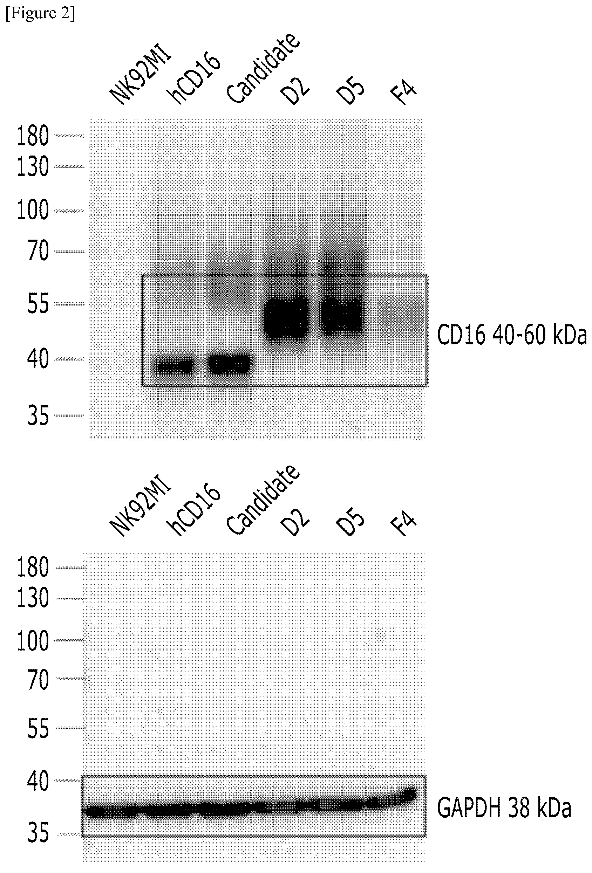

FIG. 2 shows the expression of CD16 receptor, measured by Western blot, in the intracellular domain of NK92MI cells expressing engineered receptors. Both CD16 full form and CD64/16 chimeric form receptors expressed CD16 receptors inwardly, which could be confirmed through the bands. On the other hand, no bands were observed in the NK92MI experimental group that did not express the receptors.

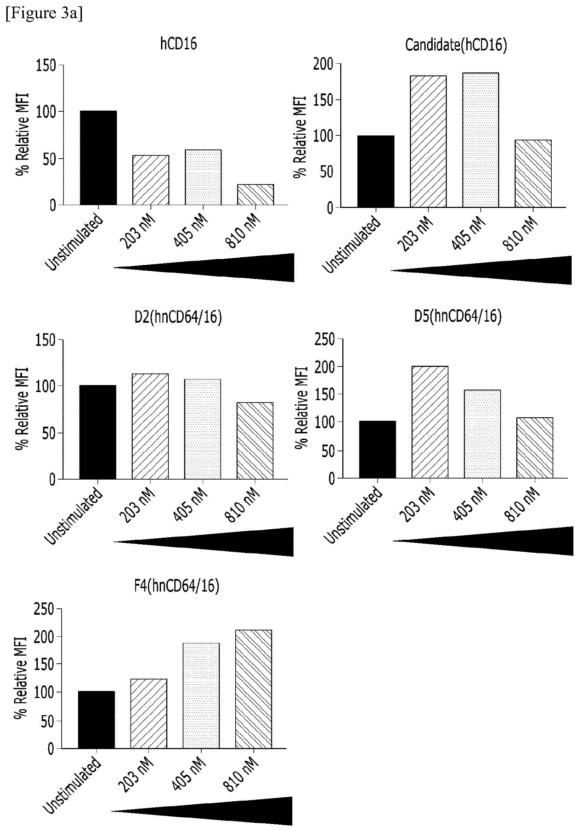

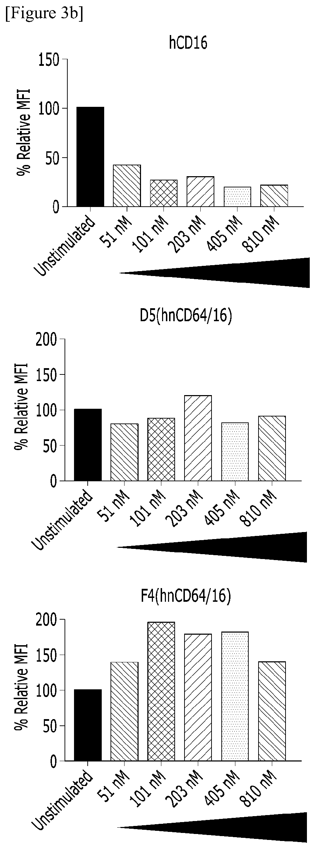

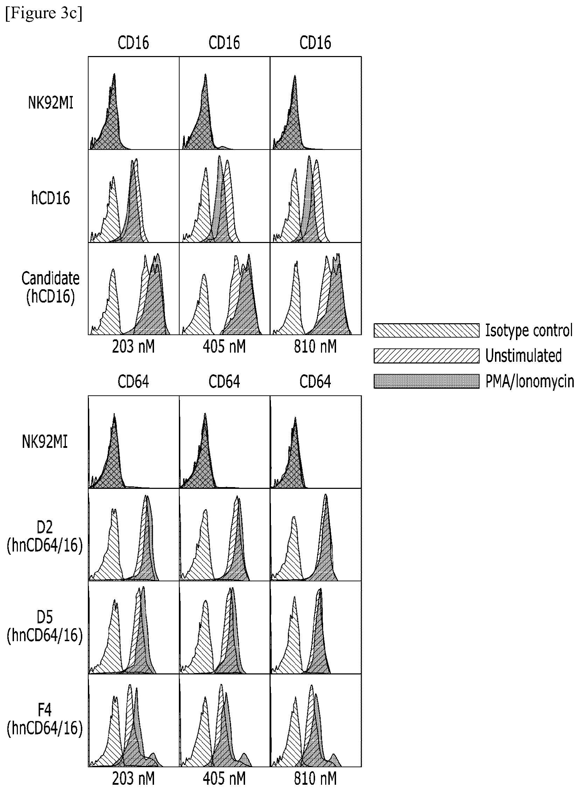

FIG. 3 shows the effect of a disintegrin and metalloproteinase 17 (ADAM17) shedding on NK92MI cells expressing engineered receptors, measured by flow cytometry, when the cells were strongly stimulated with phorbol 12-myristate 13-acetate (PMA)/Ionomycin. FIG. 3A shows the relative mean fluorescence intensity (MFI) value of receptor expression when the MFI of the unstimulated was set to 100. The control (hCD16) was cleaved at all three stimulation concentrations and thus the expression decreased, while the Candidate, D2, D5, and F4 cell lines were not cleaved and their expression rather increased or maintained. The graph in FIG. 3B shows the results of measuring the expression of the receptors by flow cytometry at five PMA/Ionomycin concentrations. The expression of the activated control was down-regulated compared to the unstimulated experimental group, while the expression of D5 and F4 was up-regulated or maintained even upon activation, supplementing the results shown in FIG 3A. FIG. 3C shows the results of FIG. 3A as a histogram.

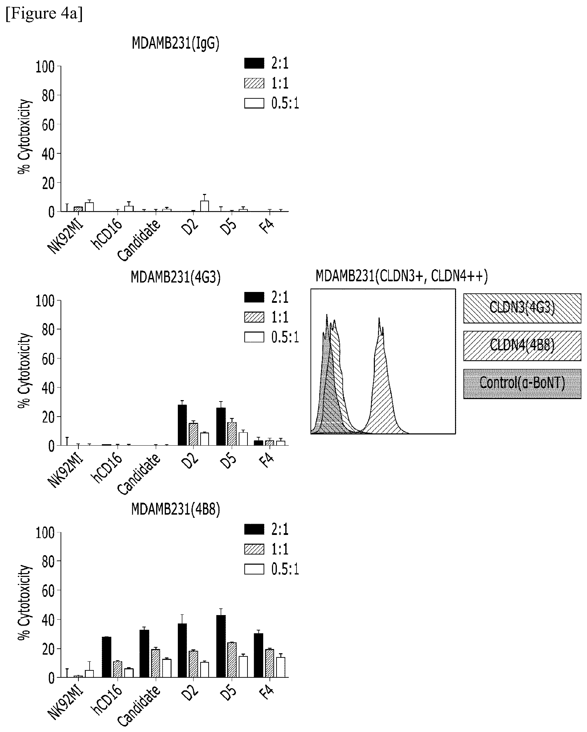

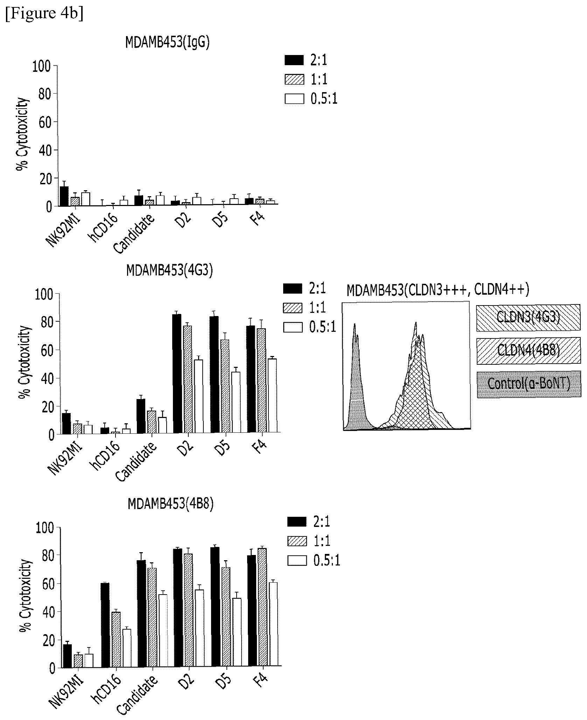

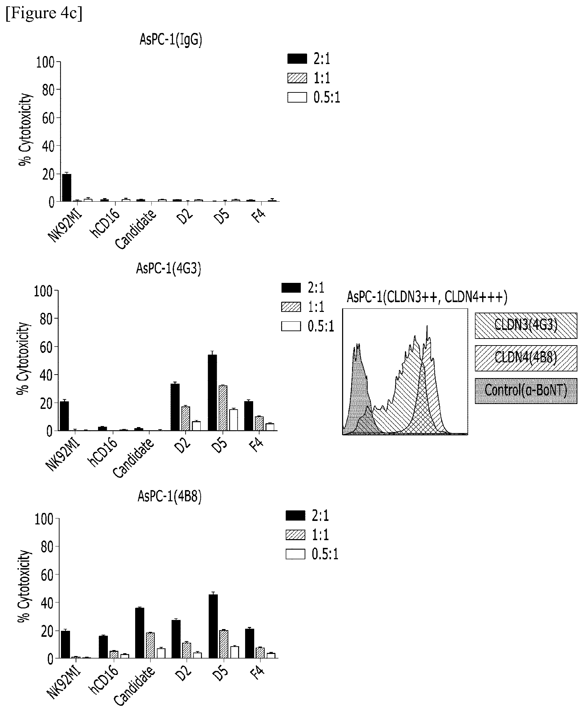

FIG. 4 shows the antibody-dependent cellular cytotoxicity (ADCC) activity of NK92MI cells expressing each engineered receptor in various cancer cell lines, measured by lactate dehydrogenase (LDH) release from target cells. LDH is a cytoplasmic enzyme released into the culture supernatant when the cell membrane is damaged during cell death, and it is commonly used as an indicator of cytotoxicity. FIGS. 4A and 4B show the high cytotoxicity of Candidate, D2, and D5 compared to NK92MI or the control, when antibodies targeting the antigen were used in MDA-MB-231 and MDA-MB-453 cell lines compared to the control IgG experimental group. FIG. 4C shows the ADCC results in AsPC-1 cell line, which exhibited resistance to lysis by NK92 cells expressing CD16 compared to other cell lines.

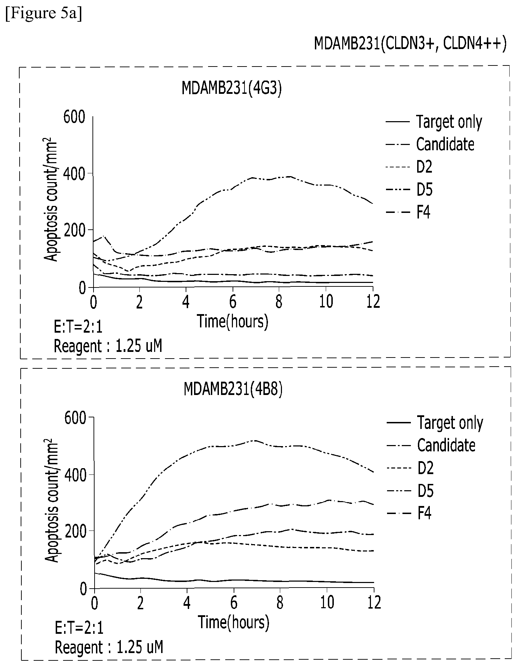

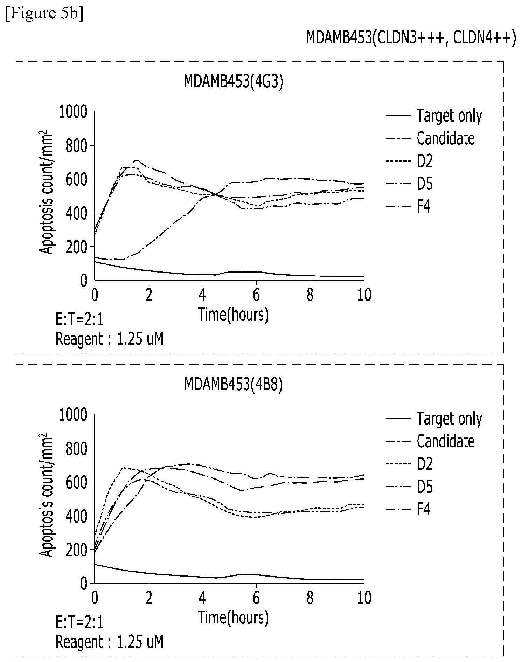

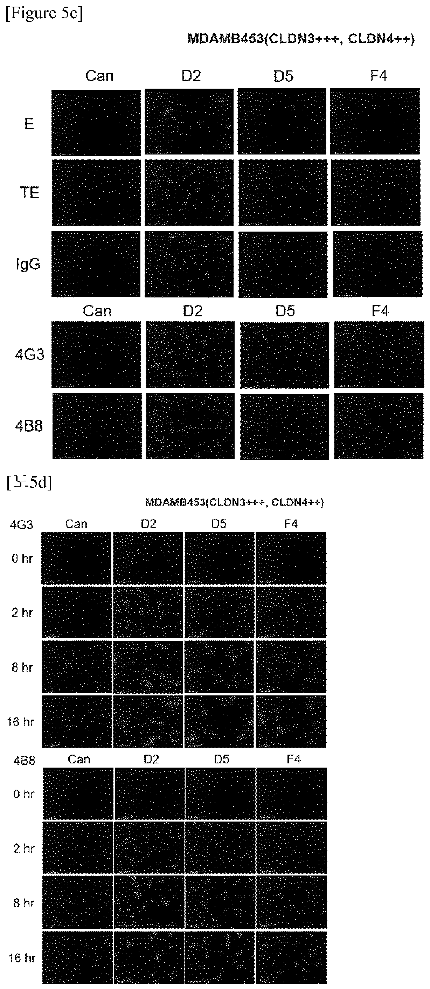

FIG. 5 shows the long-term ADCC activity of NK92MI cells expressing engineered receptors in various cancer cell lines, measured by using the IncuCyte real-time imaging equipment and Caspase-3/7 red dye. Green fluorescence was confirmed in D2, D5, and F4 NK92MI cell lines including vectors containing green fluorescent protein (GFP), and dead target cells were identified by red fluorescence. FIGS. 5A and 5B show the apoptosis count over time in the ADCC situation of MDA-MB-231 and MDA-MB-453 cell lines. FIG. 5C shows the images of each experimental group after 1.5 hours when maximum apoptosis was observed. FIG. 5D shows the changes in the images of each experimental group over time.

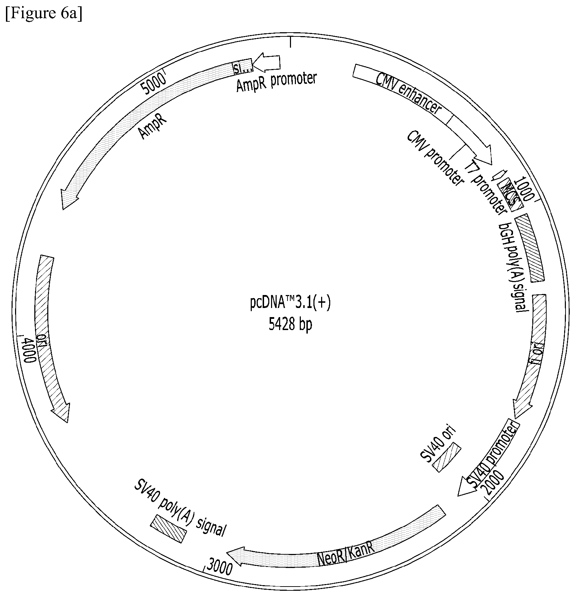

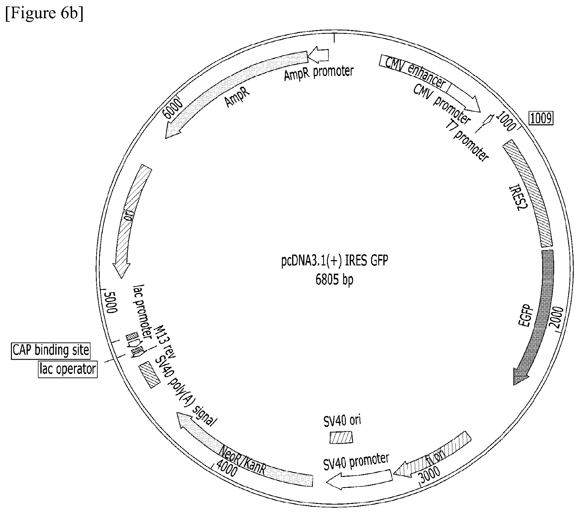

FIG. 6 shows a universal receptor vector prepared to express the recombinant Fc receptor of the present invention. FIG. 6A shows a vector used for the expression of Candidate (CD16 full form), and FIG. 6B shows a vector used for the expression of D2, D5, and F4 (CD64/16 chimera).

BEST MODE

Hereinafter, the present invention will be described in detail.

The term "domain" as used herein refer to a unit having functional or structural characteristics. The domain according to the present invention is preferably a polypeptide. This polypeptide may be a single polypeptide chain or may be a module formed by assembling parts of one or more polypeptide chains. The polypeptide may include proteinaceous parts and non-proteinaceous parts, where the non-proteinaceous parts may be, for example, a chemical cross-linker or a chemical linker such as glutaraldehyde.

The amino acid sequence included in the present specification may encompass sequences having 80% or more, preferably 90% or more, more preferably 95% or more, and most preferably 99% or more homology with the sequence, when a change is made such that a sequence difference is exhibited while maintaining the characteristics of each domain according to the present invention. The change may be any one or more selected from addition, substitution, or deletion of some amino acid residues.

The term "amino acid substitution" or "substitution" as used herein means that an amino acid at a specific position in a parent polypeptide sequence is replaced with a different amino acid.

The present invention relates to a recombinant Fc receptor.

The present invention relates to a recombinant Fc receptor including at least a part of a CD16 polypeptide including an ADAM17 cleavage site sequence corresponding to positions 196 to 198 of SEQ ID NO: 1, the recombinant Fc receptor including at least one of: i) at least one amino acid modification of substitution of serine at position 197 with proline and substitution of threonine at position 198 with serine; and ii) replacement of all or a part of an ectodomain of the CD16 polypeptide with an ectodomain of CD64.

CD16 is expressed in two different forms, namely CD16a and CD16b, which are products of two different but highly homologous genes. CD16a is a polypeptide-anchored protein, and CD16b is a glycosylphosphatidylinositol-anchored protein. As used herein, CD16 refers to the two forms of this protein that are obvious to one of ordinary skill in the art, unless otherwise inappropriate.

The CD16 polypeptide may be any CD16 polypeptide sequence known to the National Center for Biotechnology Information (NCBI) or the like, when it includes a disintegrin and metalloproteinase 17 (ADAM17) cleavage site sequence.

The recombinant Fc receptor of the present invention may include, for example, at least a part of a CD16 polypeptide having an amino acid sequence of SEQ ID NO: 1.

ADAM 17 is a protease that cleaves the ectodomain of CD16 when signaling through CD16 is activated, thereby inhibiting antibody dependent cell cytotoxicity (ADCC) activation signaling. The ADAM 17 cleavage site sequence may include, for example, a sequence corresponding to positions 196 to 198 of SEQ ID NO: 1, but is not limited thereto.

The recombinant Fc receptor of the present invention includes at least one of: i) at least one amino acid modification of substitution of serine at position 197 with proline and substitution of threonine at position 198 with serine; and ii) replacement of all or a part of an ectodomain of the CD16 polypeptide with an ectodomain of CD64.

When the recombinant Fc receptor of the present invention includes at least one amino acid modification of substitution of serine at position 197 with proline and substitution of threonine at position 198 with serine, specifically, the ADAM17 cleavage site sequence corresponding to positions 196 to 198 of SEQ ID NO: 1 of the CD16 polypeptide may be valine at position 196, proline at position 197, and threonine at position 198, or valine at position 196, proline at position 197, and serine at position 198, or valine at position 196, serine at position 197, and serine at position 198.

The recombinant Fc receptor of the present invention may have cleavage resistance to ADAM17 by including the above-described amino acid modification.

The recombinant Fc receptor of the present invention may include replacement of all or a part of an ectodomain of the CD16 polypeptide with an ectodomain of CD64.

An Fc receptor includes an ectodomain, a transmembrane domain, and an intracellular domain, and the present invention may include a part of or all ectodomain of CD64. Both CD64 and CD16 bind to the Fc region of antibodies, but CD64 has about 100- to 1,000-fold higher affinity. CD64 includes a glycoprotein α chain whose ectodomain consists of three immunoglobulin domains responsible for antibody binding, and it has been confirmed that the presence of all the three immunoglobulin domains is important for high-affinity interaction with antibodies. On the other hand, the ectodomain of CD16 has only two immunoglobulin-like domains. The presence of an extra immunoglobulin-like fold within CD64 may be a strong determinant of high Fc affinity. The recombinant Fc receptor of the present invention may also have high affinity for antibodies by simply inserting this extra immunoglobulin-like fold structure or its mutant version within CD64 into the ectodomain of CD16.

The present invention may be a fusion of the ectodomain of CD64 to any suitable transmembrane domain and intracellular binding region of CD16. In addition, it may be a fusion of three immunoglobulin domains (EC1, EC2, EC3) and a part of the hinge of CD64 with a part of the hinge, the transmembrane domain, and the intracellular binding region of CD16, but is not limited thereto, and the ectodomain of CD64 may be replaced at a suitable position corresponding to all or a part of the ectodomain of the CD16 polypeptide to improve the affinity of the CD16 polypeptide for the antibody Fc region.

The ectodomain of the CD64 may include, for example, an amino acid sequence of SEQ ID NO: 2 or 3.

In the present invention, for example, the CD16 polypeptide may include the amino acid sequence of SEQ ID NO: 1, including at least one amino acid modification of substitution of the serine at amino acid position 197 with proline and substitution of the threonine at amino acid position 198 with serine; and substitution of the amino acid at position 176 of SEQ ID NO: 1 with valine. More specifically, the recombinant Fc receptor of the present invention may include the amino acid sequence of SEQ ID NO: 4.

In addition, the present invention may be, for example, one in which the serine at amino acid position 197 is substituted with proline, and all or a part of the ectodomain of the CD16 polypeptide is replaced with an ectodomain of CD64. More specifically, the recombinant Fc receptor of the present invention may include the amino acid sequence of SEQ ID NO: 5.

In addition, the present invention may be, for example, one in which the serine at amino acid position 197 is substituted with proline, and the threonine at amino acid position 198 is substituted with, and all or a part of the ectodomain of the CD16 polypeptide is replaced with an ectodomain of CD64. More specifically, the recombinant Fc receptor of the present invention may include the amino acid sequence of SEQ ID NO: 6 or 7.

The present invention may be, for example, one in which all or a part of the ectodomain of the CD16 polypeptide is replaced with an ectodomain of CD64. More specifically, the recombinant Fc receptor of the present invention may include the amino acid sequence of SEQ ID NO: 8.

The present invention relates to a nucleic acid molecule encoding the Fc receptor.

The nucleic acid molecule has a meaning that comprehensively encompasses DNA (gDNA and cDNA) and RNA molecules, and the nucleotide, which is a basic structural unit in an nucleic acid molecule, may include not only natural nucleotides but also analogues in which sugar or base moieties are modified (Scheit, Nucleotide Analogs, John Wiley, New York (1980); Uhlman and Peyman, Chemical Reviews, 90:543-584 (1990)). The nucleic acid molecule sequence encoding the recombinant Fc receptor of the present invention may be modified. The modification may include addition, deletion, or non-conservative substitution or conservative substitution of nucleotides. As known in the relevant technical field, the nucleic acid molecule of the present invention may be incorporated into an expression vector depending on the host cell that is used to produce the recombinant Fc receptor of the present invention.

The present invention relates to an expression vector including the nucleic acid molecule.

The expression vector may include an expression regulatory sequence operably linked to the nucleic acid molecule.

The term "vector" as used herein refers to a nucleic acid molecule capable of carrying another nucleic acid to which it is linked. For example, the vector includes a plasmid, that is, a circular double-stranded piece of DNA into which an additional DNA segment may be ligated. In addition, the vector includes a viral vector, that is, a vector that allows an additional DNA segment to be ligated into the viral genome. The vector may autonomously replicate within a host cell into which it is introduced. For example, it may be a bacterial vector having a bacterial origin of replication and an episomal mammalian vector. In addition, the vector (e.g., a non-episomal mammalian vector) may be integrated into the genome of a host cell by being introduced into the host cell, thereby replicating together with the host genome. Furthermore, any vector may express a gene operably linked to the vector.

The term "expression regulatory sequence" used in the present invention refers to a polynucleotide sequence necessary for ligation of a coding sequence to express and process the coding sequence. The expression regulatory sequence includes appropriate transcription initiation, termination, promoter, and enhancer sequences, efficient RNA processing signals such as splicing and polyadenylation signals, sequences that stabilize cytoplasmic mRNA, sequences that increase translation efficiency (i.e., Kozak consensus sequences), and sequences that enhance protein stability, and, when desired, sequences that promote protein secretion. The nature of such expression regulatory sequences varies depending on the host organism. Prokaryotes generally include a promoter, a ribosome binding site, and a transcription termination sequence as such expression regulatory sequences, and eukaryotes include a promoter and a transcription termination sequence as such expression regulatory sequences. In the present invention, the expression regulatory sequence includes at least all components whose presence is essential for the expression and processing process, and may include additional components whose presence is advantageous, such as a leader sequence and a fusion partner sequence.

The present invention relates to a host cell transformed with the expression vector.

The term "host cell" as used in the present invention refers to a cell that may be used to express a nucleic acid, for example, a nucleic acid of the present invention. Typically, a host cell is a cultured cell that may be transformed or transfected with a polypeptide-encoding nucleic acid, and then the nucleic acid may be expressed in the host cell.

The host cell of the present invention may be, for example, a mammalian immune cell, stem cell, or cell differentiated from a stem cell.

The immune cells may be, for example, natural killer cells (NK cells), T cells, B cells, NKT cells, neutrophils, eosinophils, basophils, mast cells, monocytes, dendritic cells, or macrophages. In addition, the immune cells may be cytotoxic effector cells. Here, the cytotoxic effector cells may be NK cells, cytotoxic T cells, macrophages, monocytes, neutrophils, and eosinophils. The cells may preferably be NK cells or cytotoxic T cells. Among these, T cells have the characteristic of interacting with the major histocompatibility complex (MHC) and peptides loaded into the MHC through the T cell receptor (hereinafter referred to as TCR), and at this time, the T cells attack a cell expressing an MHC recognized as foreign by the TCR. On the other hand, NK cells do not include a TCR, so they have the advantage of not causing a graft-versus-host disease (GVHD), in which transplanted cells attack the target's cells, when administered to the target for a therapeutic purpose.

The stem cells may be, for example, embryonic stem cells, adult stem cells, or induced pluripotent stem cells.

Since immune cells are often not easy to proliferate, transforming stem cells with high proliferative potential may be advantageous in terms of single clone proliferation.

The host cells may be any one of autologous, allogeneic, or xenogeneic cells, and xenogeneic cells are preferably obtained from a mammal. Preferably, the host cells may be selected from among 1) autologous or 2) allogeneic cells in which genes related to immune response regulation have been manipulated, in order to reduce the possibility of immune rejection. The genes related to immune response regulation may include genes related to attack of host cells by transplanted cells and/or attack of transplanted cells by host cells. Genes related to the attack of the host cells by the transplanted cells include, for example, TCRs that recognize MHC, and may be manipulated to reduce or eliminate their expression. In addition, when non-classical MHCs such as HLA-G or HLA-E are expressed, there may be an effect of protecting the transplanted cells from the host cells.

The host cells may be ones in which the expression of TAP1, TAP2, TAPBP, NLRC5, CIITA, RFXANK, RFX5, RFXAP, and the like, which are related to the expression of MHC, has been reduced or eliminated.

Specifically, the host cells may be ones that knock out the genes required for antigen presentation of MHC-1 of the cells, and at the same, insert a recombinase recognition sequence into the gene region of the cells and prepare it as a landing pad so that the cells are safely transformed, but are not limited thereto.

The gene region may include not only exon regions, but also all regions including enhancers, promoters, and introns that are related to the expression regulation of the genes.

By reducing or eliminating the expression of the genes, the expression of MHC, which may be recognized as a non-self molecule by the host TCR, may be reduced or eliminated.

Cells in which the genes related to immune response regulation are engineered as described above may be prepared as off-the-shelf in advance, which may have advantages in terms of time and cost, compared to autologous cells that must be transduced, amplified, screened, and tested for activity for each new patient.

The present invention can effectively treat cancer or viral infection by simultaneously or sequentially administering the host cell and antibody to a subject.

The antibody may be selected from antibodies specific to one or more antigens, one or more monoclonal antibodies combined, or a combination thereof. The antibody may be selected from, for example, i) an immunoglobulin selected from IgG1, IgG2, IgG3 and IgG4; ii) a native antibody fragment such as Fv, Fab, Fab', F(ab')2, VHH, and VNAR.; iii) an engineered antibody such as scFv, dsFv, ds-scFv, (scFv)2, diabody, triabody, tetrabody, and pentabody, and at this time, the antibody preferably includes a protein fragment derived from IgG which is involved in binding of the Fc binding domain of CD16 and CD64 to IgG, for example, a protein fragment including an Fc domain or a part thereof and optionally a hinge domain or a part thereof. The protein fragment derived from the IgG may be preferably derived from IgG1, IgG3, or IgG4 among IgGs, and more preferably derived from IgG1 or IgG3. The affinity of the Fcγ receptor to Fc is known to be in the order of IgG3>IgG1>IgG4, and in particular, IgG1 and IgG3 are known to serve as potent activators of antibody-dependent effector functions. Although IgG3 may activate such effector functions most strongly, it is known that IgG1 is preferred for the purpose of inducing therapeutic cytotoxicity because its half-life is short in the body. The antibody may include one antigen-binding domain (monovalent) or may include more than one antigen-binding domain (multivalent) . The antibody may be monoclonal or polyclonal. The antibody may be a naturally occurring antibody isolated or purified from an animal such as a mouse, a rabbit, a goat, a horse, a chicken, a hamster, or a human, or a synthetic antibody designed and genetically synthesized with reference to the sequence characteristics of a naturally occurring antibody. The antibody may be an engineered antibody such as a humanized antibody or a chimeric antibody. The antibody may be in a monomeric or polymeric form. The antibody may include one or more of chemical modifications, such as addition of a disulfide bond, glycosylation, an amino acid with a modified side chain, oxidation of methionine, deamidation of asparagine or glutamine, γ-carboxylation, β-hydroxylation, and sulfation. The antibody may have any level of affinity or avidity for the target antigen. The antibody may be any one selected from mouse, rabbit, human, humanized, or chimeric proteins.

The subject may be an animal, preferably a mammal, particularly an animal including a human, and may also be a cell, tissue, organ, and the like derived from the animal. In addition, the subject may be a patient in need of the treatment.

The present invention relates to a pharmaceutical composition for treating cancer or viral infection, including the host cell.

The cancer is not particularly limited, and may be, for example, adenocarcinoma, breast cancer, pancreatic cancer, blood cancer, ovarian cancer, colon cancer, bladder cancer, lung cancer, liver cancer, stomach cancer, esophageal cancer, prostate cancer, uterine cancer, cervical cancer, melanoma, colon cancer, kidney cancer, or metastatic pleural tumor.

The virus is not limited to a specific type of virus, and may be a double stranded DNA (dsDNA) -based virus, a single stranded (ssDNA)-based virus, or an RNA virus. Specifically, it may be adenovirus, herpesvirus, mamavirus, parvovirus, reovirus, rotavirus, or retrovirus, and is not particularly limited as long as the composition of the present invention exhibits antiviral activity.

The term "treatment" as used herein refers to an activity that ameliorates or favorably alters symptoms caused by cancer or viral infection.

The composition may include a pharmaceutically acceptable carrier.

The pharmaceutically acceptable carrier refers to any and all physiologically compatible solvents, dispersion media, coatings, antibacterial and antifungal agents, isotonic agents, and absorption retardation agents, and the like. Examples of pharmaceutically acceptable carriers include water, saline, phosphate buffered saline, dextrose, glycerol, and ethanol, and mixtures thereof. In many cases, it will be preferable to include isotonic agents, such as sugars, polyhydric alcohols such as mannitol and sorbitol, or sodium chloride, in the composition.

The composition of the present invention may take various forms, for example, liquid, semisolid, and solid dosage forms, for example, liquid solutions (e.g., injectable and infusible solutions), dispersions or suspensions, tablets, pills, powder, liposomes, and suppositories. The preferred form depends on the intended administration route and therapeutic method. The composition of the present invention may take the form of a suspension, a solution or an emulsion in an oleaginous or aqueous vehicle, and may contain formulation agents, for example, suspending agents, stabilizing agents, and/or dispersing agents. Alternatively, the active ingredient may be present before use in a powder form for use with a suitable vehicle, for example, a sterile, pyrogen-free substance.

The pharmaceutical composition of the present invention may be formulated and used in the form of oral formulations such as powder, granules, tablets, capsules, suspensions, emulsions, syrup, and aerosols, external preparations, suppositories, or sterile injection solutions according to the conventional methods. Specifically, when formulated, it may be prepared using diluents or excipients such as fillers, weighting agents, binders, wetting agents, disintegrating agents, and surfactants that are commonly used. Solid preparations for oral administration include tablets, pills, powder, granules, and capsules, but are not limited thereto. In addition to simple excipients, lubricants such as magnesium stearate and talc may also be used. In addition to liquids and liquid paraffin for oral administration, it may be prepared by adding various excipients such as wetting agents, sweeteners, fragrances, and preservatives. Preparations for parenteral administration may include sterile aqueous solutions, non-aqueous solvents, suspensions, emulsions, lyophilized preparations, and suppositories. Non-aqueous solvents and suspensions may include propylene glycol, polyethylene glycol, vegetable oils such as olive oil, and injectable esters such as ethyl oleate. As a base for suppositories, Witepsol, Macrogol, Tween 61, cacao oil, laurel oil, glycerol, gelatin, or the like may be used

The appropriate dosage of the pharmaceutical composition of the present invention varies depending on the patient's condition and weight, the severity of the disease, the drug form, and the time, but may be appropriately selected by a person of ordinary skill in the art.

The composition may further include an antibody, and the antibody is as described above.

Hereinafter, the present invention will be described in detail by way of examples in order to specifically explain the present invention.

Manufacturing Example: Production of universal Fc receptor NK cells binding to the Fc portion of antibodies

1. Production of expression vectors

A construct of universal Fc receptor was ordered to Bionics for synthesis in the puC vector, and the sequence portion was cloned to express Candidate (SEQ ID NO: 4) in pcDNA3.1(+) vector (FIG. 6A) and D2 (SEQ ID NO: 6), D5 (SEQ ID NO: 5), and F4 (SEQ ID NO: 8) in pcDNA3.1(+) internal ribosome entry site (IRES) green fluorescent protein (GFP) vector (FIG. 6B). The vector of Candidate included sequence changes in F176V, S197P, and T198S in the sequence of human CD16a. D2, D5, and F4 included CD64 receptor sequences up to S1, S2, EC1, EC2, EC3, and a part of the hinge, and the remaining hinge part, TM, and IC included the sequence of hCD16a. The D2, D5, and F4 sequences included a sequence for enhanced green fluorescent protein (EGFP) to confirm expression.

The control group used hCD16 containing a sequence change at F176V in human CD16a (SEQ ID NO: 9).

2. Vector transfection and cell line construction

The produced vector was transfected into NK92MI cells, a cell line derived from NK cell non-Hodgkin's lymphoma, to confirm expression. The transfection was performed via electroporation, and the kit used was the Neon transfection system kit from Thermo Fisher. After optimizing the NK92MI cell line transfection conditions provided by Thermo (1200 V, 20 ms/3 pulses), the experiment was performed using the medium added at 37 °C and 5% $CO_2$ conditions, and after a stabilization period of two to three weeks, cells expressing CD16 in Candidate and cells expressing GFP in D2, D5, and F4 were primarily sorted using FITC anti-human CD16 antibody (Biolegend) through BD FACS Aria III. Then, after a stabilization period of two to three weeks, Universal-NK cell lines were constructed by performing secondary sorting in the same manner or treating the cells with G418 Sulfate (Enzo) for two weeks.

Experimental Example 1. Confirmation of vector expression (flow cytometry)

Using the cells of Manufacturing Example, the receptor expression of the EC domain was confirmed by flow cytometry. Cells were treated with mouse IgG1 kappa Isotype Control, allophycocyanin (APC, Invitrogen), anti-CD16-APC, and anti-CD64-APC antibodies, stained, washed with a fluorescence-activated single cell sorting (FACS) buffer after 45 minutes, and analyzed with BD FACSLyric. Through this experiment, the expression of the CD16 receptor of the EC domain was confirmed in the control (hCD16) and Candidate, and the expression of the CD64 receptor was confirmed in D2, D5, and F4 (FIG. 1).

Experimental Example 2. Confirmation of vector expression (Western blot)

Using the cells of Manufacturing Example, the expression of CD16 receptor of the IC domain was confirmed by Western blot. After cell lysis in the radioimmunoprecipitation assay (RIPA) buffer for one hour, cells were spun down at 14,000 rpm to recover only the protein, and then the concentration was confirmed through a bicinchoninic acid (BCA) assay (Pierce, Themo Fisher). After confirming the concentration, about 10 $\mu$g of protein was transferred to a polyvinylidene fluoride (PVDF) membrane after sodium dodecyl sulfate-polyacrylamide gel electrophoresis (SDS-PAGE) and blocked with 5% skim milk at RT for one hour. The protein was allowed to react with anti-CD16-antibody (Abcam) that binds to CD16 C-terminus at a ratio of 1:1000 at 4 °C overnight. After the reaction, bands were confirmed with Image Quant 800. As a result, it was confirmed through the bands that both CD16 full form NK cells (hCD16, Candidate) and CD64/16 chimeric form NK cells (D2, D5, F4) expressed CD16 receptor inwardly. On the other hand, no band was observed in the NK92MI experimental group that did not express the receptor (FIG. 2).

Experimental Example 3: Confirmation of receptor cleavage by ADAM17 through flow cytometry

Using the cells of Manufacturing Example, receptor cleavage by ADAM17 was tested. First, the expression level of CD16 or CD64 was examined through flow cytometry to confirm whether ADAM17 shedding and cleavage decreased receptor expression when the Universal-NK cells were strongly stimulated with phorbol 12-myristate 13-acetate (PMA)/Ionomycin. Cells were treated with PMA/Ionomycin cocktail (Biolegend) at three different concentrations, harvested after four hours, stained with each of the antibodies (Biolegend) recognizing CD16 and CD64, and analyzed using BD FACSLyric. In the control group, cleavage occurred at all the three stimulation concentrations and decreased the expression, whereas the expression increased or was maintained in Candidate, D2, D5, and F4 cell lines in which cleavage did not occur (FIGS. 3A and 3C). In addition, receptor expression was measured by flow cytometry at five different PMA/Ionomycin concentrations. The expression of the activated control was down-regulated compared to the unstimulated experimental group, while the expression of D5 and F4 was up-regulated or maintained even upon activation (FIG. 3B).

Experimental Example 4: Confirmation of the efficacy of universal Fc receptor

After confirming the expression of the vector, antibody dependent cell cytotoxicity (ADCC) experiments were conducted with MDA-MB-231, MDA-MB-453, and AsPC-1 cell lines to confirm the efficacy of Universal-NK cells.

4-1. Confirmation of ADCC activity through lactate dehydrogenase (LDH) release

To determine the LDH release from target cells due to the ADCC mechanism by combining target cells, antibodies, and Universal-NK cells, the CytoTox 96® Non-Radioactive Cytotoxicity Assay kit (Promega) was used. The target cells used were MDA-MB-231, MDA-MB-453, and AsPC-1 cell lines. After seeding the target cells in a 96-well plate and stabilizing for one day, 1 $\mu$g/ml antibodies and Universal-NK cells were added at three ratios: E:T=2:1, 1:1, and 0.5:1. After four hours, the 96-well plate was spun down, and only the cell supernatant was collected and placed in a new 96-well plate for each experimental group. After conducting the experiment according to the assay kit protocol, the absorbance was measured at 490 nm using a multireader. The percent cytotoxicity (% cytotoxicity) was calculated by the method shown below.

$$\% \text{ Cytotoxicity} = \frac{\text{Experimental} - \text{Effector Spontaneous} - \text{Target Spontaneous}}{\text{Target Maximum} - \text{Target Spontaneous}} \times 100$$

The experimental results showed that Candidate, D2, and D5 exhibited higher cytotoxicity compared to NK92MI or the control group, when antibodies targeting the antigen were used in all cell lines compared to the control IgG experimental group (FIGS. 4A to 4C) .

4-2. Confirmation of long-term ADCC activity through Caspase-3/7 staining

To investigate the apoptosis of target cells due to the ADCC mechanism by combining target cells, antibodies, and Universal-NK cells, IncuCyte real-time imaging equipment and Caspase-3/7 apoptosis red dye (Sartorius AG) were used. The red dye binds to the motif of the DEVD peptide recognized by Caspase 3/7 of the target cells. The target cells used were MDA-MB-231 and MDA-MB-453 cell lines. There is no change in healthy cells, but in apoptotic cells, activated Caspase cleaves the DEVD recognition motif and enters the nucleus, causing DNA to fluoresce red, so that dead cells are quantified in real time. Target cells were seeded in 96-well plates, and after stabilization for one day, treated with antibodies and the red dye, and Universal-NK cells were added at a ratio of E:T=2:1. In D2, D5, and F4 NK92MI cell lines containing vectors including GFP, green fluorescence was confirmed, and dead target cells were confirmed with red fluorescence. The experimental results showed that D2 and D5 exhibited higher cytotoxicity compared to Candidate in both cell lines (FIGS. 5A to 5D).

The above description of the present invention is for illustrative purposes, and those skilled in the art will understand that it can be easily modified into other specific forms without changing the technical idea or essential features of the present invention. Therefore, it should be understood that the above-described examples are exemplary in all respects and not limiting. For example, each component described as a single type may be implemented in a distributed manner, and likewise, components described as distributed may be implemented in a combined form.

The scope of the present invention is indicated by the claims described below, and all changes or modifications derived from the meaning and scope of the claims and the equivalent concepts thereof should be construed as being included in the scope of the present invention.

**Claims**

1. A recombinant Fc receptor comprising at least a part of a CD16 polypeptide including an ADAM17 cleavage site sequence corresponding to positions 196 to 198 of SEQ ID NO: 1, the recombinant Fc receptor comprising at least one of:

   i) at least one amino acid modification of substitution of serine at position 197 with proline and substitution of threonine at position 198 with serine; and
   ii) replacement of all or a part of an ectodomain of the CD16 polypeptide with an ectodomain of CD64.

2. The recombinant Fc receptor according to claim 1, wherein the CD16 polypeptide includes an amino acid sequence of SEQ ID NO: 1, and the recombinant Fc receptor includes: at least one amino acid modification of substitution of the serine at amino acid position 197 with proline and substitution of the threonine at amino acid position 198 with serine; and substitution of the amino acid at position 176 of SEQ ID NO: 1 with valine.

3. The recombinant Fc receptor according to claim 1, wherein the serine at amino acid position 197 is substituted with proline, and all or a part of the ectodomain of the CD16 polypeptide is replaced with an ectodomain of CD64.

4. The recombinant Fc receptor according to claim 1, wherein the serine at amino acid position 197 is substituted with proline, the threonine at amino acid position 198 is substituted with serine, and all or a part of the ectodomain of the CD16 polypeptide is replaced with the ectodomain of CD64.

5. The recombinant Fc receptor according to claim 1, wherein all or a part of the ectodomain of the CD16 polypeptide is replaced with the ectodomain of CD64.

6. The recombinant Fc receptor according to claim 1, wherein the ectodomain of CD64 includes an amino acid sequence of SEQ ID NO: 2 or 3.

7. The recombinant Fc receptor according to claim 1, wherein the recombinant Fc receptor includes any one amino acid sequence of SEQ ID NOs: 4 to 8.

8. A nucleic acid molecule encoding the Fc receptor according to any one of claims 1 to 7.

9. An expression vector comprising the nucleic acid molecule according to claim 8.

10. A host cell transformed with the expression vector according to claim 9.

11. The host cell according to claim 10, wherein the host cell is a mammalian immune cell, stem cell, or cell differentiated from a stem cell.

12. A pharmaceutical composition for treating cancer, comprising the host cell according to claim 10.

13. A pharmaceutical composition for treating viral infection, comprising the host cell according to claim 10.

[Figure 1]

[Figure 2]

CD16 40-60 kDa

GAPDH 38 kDa

[Figure 3a]

hCD16

Candidate(hCD16)

D2(hnCD64/16)

D5(hnCD64/16)

F4(hnCD64/16)

[Figure 3b]

hCD16

D5(hnCD64/16)

F4(hnCD64/16)

[Figure 3c]

[Figure 4a]

MDAMB231(IgG)

MDAMB231(4G3)

MDAMB231(CLDN3+, CLDN4++)

CLDN3(4G3)

CLDN4(4B8)

Control(α-BoNT)

MDAMB231(4B8)

[Figure 4b]

MDAMB453(IgG)

MDAMB453(4G3)

MDAMB453(CLDN3+++, CLDN4++)

MDAMB453(4B8)

[Figure 4c]

AsPC-1(IgG)

AsPC-1(4G3)

AsPC-1(4B8)

[Figure 5a]

MDAMB231(CLDN3+, CLDN4++)

MDAMB231(4G3)

— Target only
—·— Candidate
----- D2
—··— D5
— — F4

E:T=2:1
Reagent : 1.25 uM

MDAMB231(4B8)

— Target only
—·— Candidate
----- D2
—··— D5
— — F4

E:T=2:1
Reagent : 1.25 uM

[Figure 5b]

MDAMB453(CLDN3+++, CLDN4++)

MDAMB453(4G3)

Target only, Candidate, D2, D5, F4

E:T=2:1
Reagent : 1.25 uM

MDAMB453(4B8)

Target only, Candidate, D2, D5, F4

E:T=2:1
Reagent : 1.25 uM

[Figure 5c]

MDAMB453(CLDN3+++, CLDN4++)

[도5d]

MDAMB453(CLDN3+++, CLDN4++)

[Figure 6a]

[Figure 6b]

pcDNA3.1(+) IRES GFP
6805 bp

CMV enhancer
CMV promoter
T7 promoter
1000
1009
IRES2
EGFP
2000
N ori
3000
SV40 ori
SV40 promoter
NeoR/KanR
4000
SV40 poly(A) signal
M13 rev
lac promoter
5000
CAP binding site
lac operator
f1 ori
AmpR
AmpR promoter
6000

## INTERNATIONAL SEARCH REPORT

| | International application No. |
|---|---|
| | **PCT/KR2023/004638** |

| A. | CLASSIFICATION OF SUBJECT MATTER |
|---|---|

**C07K 14/735**(2006.01)i; **A61K 35/15**(2015.01)i; **A61P 35/00**(2006.01)i; **A61P 31/12**(2006.01)i; **A61K 38/00**(2006.01)i

According to International Patent Classification (IPC) or to both national classification and IPC

| B. | FIELDS SEARCHED |
|---|---|

Minimum documentation searched (classification system followed by classification symbols)

C07K 14/735(2006.01); A61K 45/06(2006.01); C12N 15/12(2006.01); C12N 15/90(2006.01); C12N 5/0783(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: ADAM17, 절단부위(cleavage site), CD16 폴리펩타이드(CD16 polypeptide), Fc 수용체(Fc receptor), 항체(antibody), S197P, T198S, 치환(substitution), 아미노산 변형(amino acid modification), CD64, 세포외 결합영역(ectodomain)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | US 2021-0332103 A1 (REGENTS OF THE UNIVERSITY OF MINNESOTA) 28 October 2021 (2021-10-28)<br>See abstract, claims 1-9 and 13-15, paragraphs [0005], [0015]-[0016], [0055]-[0056] and [0071], and SEQ ID NO: 3. | 1-2,8-13 |
| Y | | 3 |
| A | | 4-7 |
| X | KR 10-2016-0105882 A (1GLOBE BIOMEDICAL CO., LTD.) 07 September 2016 (2016-09-07)<br>See claims 1, 12-13, 20 and 25, paragraphs [0022]-[0023], [0025], [0051], [0054]-[0059], [0064]-[0066] and [0072], SEQ ID NO: 4, and figure 11. | 1,5-13 |
| Y | | 3 |
| A | US 10927346 B2 (FATE THERAPEUTICS, INC.) 23 February 2021 (2021-02-23)<br>See entire document. | 1-13 |

| ✓ Further documents are listed in the continuation of Box C. | ✓ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **17 July 2023** | **18 July 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2023/004638** |

### C. DOCUMENTS CONSIDERED TO BE RELEVANT

| Category* | Citation of document, with indication, where appropriate, of the relevant passages | Relevant to claim No. |
|---|---|---|
| A | NCBI. Genbank accession no. CAA36870.1 (07 October 2008).<br>  See entire document. | 1-13 |
| A | NCBI. Genbank accession no. AAX42508.1 (29 March 2005).<br>  See entire document. | 1-13 |
| A | EP 1734119 A2 (APPLIED RESEARCH SYSTEM ARS HOLDING N.V.) 20 December 2006<br>(2006-12-20)<br>  See entire document. | 1-13 |

Form PCT/ISA/210 (second sheet) (July 2022)

## INTERNATIONAL SEARCH REPORT

| International application No. |
| --- |
| **PCT/KR2023/004638** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT
### Information on patent family members

| International application No. |
| --- |
| **PCT/KR2023/004638** |

| Patent document cited in search report | | | Publication date (day/month/year) | Patent family member(s) | | | Publication date (day/month/year) |
| --- | --- | --- | --- | --- | --- | --- | --- |
| US | 2021-0332103 | A1 | 28 October 2021 | CN | 106715467 | A | 24 May 2017 |
| | | | | CN | 106715467 | B | 27 July 2021 |
| | | | | CN | 113699159 | A | 26 November 2021 |
| | | | | EP | 3122769 | A1 | 01 February 2017 |
| | | | | EP | 3122769 | B1 | 22 February 2023 |
| | | | | JP | 2017-511135 | A | 20 April 2017 |
| | | | | JP | 2021-035390 | A | 04 March 2021 |
| | | | | JP | 2022-121484 | A | 19 August 2022 |
| | | | | JP | 6795399 | B2 | 02 December 2020 |
| | | | | JP | 7091423 | B2 | 27 June 2022 |
| | | | | US | 10464989 | B2 | 05 November 2019 |
| | | | | US | 11370825 | B2 | 28 June 2022 |
| | | | | US | 2017-0174743 | A1 | 22 June 2017 |
| | | | | US | 2020-0017570 | A1 | 16 January 2020 |
| | | | | WO | 2015-148926 | A1 | 01 October 2015 |
| KR | 10-2016-0105882 | A | 07 September 2016 | CN | 106062183 | A | 26 October 2016 |
| | | | | CN | 111440813 | A | 24 July 2020 |
| | | | | EP | 3092304 | A2 | 16 November 2016 |
| | | | | JP | 2017-503510 | A | 02 February 2017 |
| | | | | US | 10526392 | B2 | 07 January 2020 |
| | | | | US | 2016-0355566 | A1 | 08 December 2016 |
| | | | | US | 2020-0255493 | A1 | 13 August 2020 |
| | | | | WO | 2015-106043 | A2 | 16 July 2015 |
| | | | | WO | 2015-106043 | A3 | 15 October 2015 |
| US | 10927346 | B2 | 23 February 2021 | CN | 111279336 | A | 12 June 2020 |
| | | | | CN | 111542594 | A | 14 August 2020 |
| | | | | CN | 111954715 | A | 17 November 2020 |
| | | | | EP | 3611000 | A1 | 19 February 2020 |
| | | | | EP | 3619627 | A1 | 11 March 2020 |
| | | | | EP | 3619627 | A4 | 26 August 2020 |
| | | | | EP | 3643462 | A1 | 29 April 2020 |
| | | | | EP | 3643462 | B1 | 25 January 2023 |
| | | | | EP | 3728563 | A1 | 28 October 2020 |
| | | | | EP | 3728563 | A4 | 10 November 2021 |
| | | | | EP | 3775228 | A1 | 17 February 2021 |
| | | | | EP | 3775228 | A4 | 23 February 2022 |
| | | | | EP | 3844703 | A1 | 07 July 2021 |
| | | | | EP | 3844703 | A4 | 03 November 2021 |
| | | | | EP | 4083192 | A1 | 02 November 2022 |
| | | | | JP | 2020-523716 | A | 06 August 2020 |
| | | | | JP | 2021-152931 | A | 30 September 2021 |
| | | | | JP | 2021-506236 | A | 22 February 2021 |
| | | | | JP | 2021-519061 | A | 10 August 2021 |
| | | | | KR | 10-2020-0015517 | A | 12 February 2020 |
| | | | | KR | 10-2020-0102454 | A | 31 August 2020 |
| | | | | KR | 10-2020-0127250 | A | 10 November 2020 |
| | | | | US | 10002396 | B2 | 19 June 2018 |
| | | | | US | 10121186 | B2 | 06 November 2018 |
| | | | | US | 10152756 | B2 | 11 December 2018 |
| | | | | US | 10332170 | B2 | 25 June 2019 |

Form PCT/ISA/210 (patent family annex) (July 2022)

**INTERNATIONAL SEARCH REPORT**
Information on patent family members

International application No.

**PCT/KR2023/004638**

| Patent document cited in search report | Publication date (day/month/year) | Patent family member(s) | | | Publication date (day/month/year) |
|---|---|---|---|---|---|
| | | US | 10366429 | B2 | 30 July 2019 |
| | | US | 10497037 | B2 | 03 December 2019 |
| | | US | 10504193 | B2 | 10 December 2019 |
| | | US | 10511580 | B2 | 17 December 2019 |
| | | US | 10621653 | B2 | 14 April 2020 |
| | | US | 10643266 | B2 | 05 May 2020 |
| | | US | 10650441 | B1 | 12 May 2020 |
| | | US | 10650443 | B2 | 12 May 2020 |
| | | US | 10726472 | B2 | 28 July 2020 |
| | | US | 10769717 | B2 | 08 September 2020 |
| | | US | 10825079 | B2 | 03 November 2020 |
| | | US | 10832310 | B2 | 10 November 2020 |
| | | US | 10977716 | B2 | 13 April 2021 |
| | | US | 11004139 | B2 | 11 May 2021 |
| | | US | 11074640 | B2 | 27 July 2021 |
| | | US | 11080777 | B2 | 03 August 2021 |
| | | US | 11244377 | B2 | 08 February 2022 |
| | | US | 11250493 | B2 | 15 February 2022 |
| | | US | 11282131 | B2 | 22 March 2022 |
| | | US | 11365394 | B2 | 21 June 2022 |
| | | US | 11461828 | B2 | 04 October 2022 |
| | | US | 11468497 | B2 | 11 October 2022 |
| | | US | 2015-0277681 | A1 | 01 October 2015 |
| | | US | 2015-0278922 | A1 | 01 October 2015 |
| | | US | 2015-0348153 | A1 | 03 December 2015 |
| | | US | 2016-0005091 | A1 | 07 January 2016 |
| | | US | 2016-0005095 | A1 | 07 January 2016 |
| | | US | 2016-0155171 | A1 | 02 June 2016 |
| | | US | 2016-0155173 | A1 | 02 June 2016 |
| | | US | 2016-0155174 | A1 | 02 June 2016 |
| | | US | 2016-0155175 | A1 | 02 June 2016 |
| | | US | 2016-0155176 | A1 | 02 June 2016 |
| | | US | 2016-0155177 | A1 | 02 June 2016 |
| | | US | 2016-0155180 | A1 | 02 June 2016 |
| | | US | 2016-0155184 | A1 | 02 June 2016 |
| | | US | 2016-0379213 | A1 | 29 December 2016 |
| | | US | 2016-0379298 | A1 | 29 December 2016 |
| | | US | 2017-0004588 | A1 | 05 January 2017 |
| | | US | 2017-0236196 | A1 | 17 August 2017 |
| | | US | 2017-0256000 | A1 | 07 September 2017 |
| | | US | 2017-0256001 | A1 | 07 September 2017 |
| | | US | 2017-0256003 | A1 | 07 September 2017 |
| | | US | 2017-0345105 | A1 | 30 November 2017 |
| | | US | 2018-0019984 | A1 | 18 January 2018 |
| | | US | 2018-0025442 | A1 | 25 January 2018 |
| | | US | 2018-0232817 | A1 | 16 August 2018 |
| | | US | 2019-0007381 | A1 | 03 January 2019 |
| | | US | 2019-0141021 | A1 | 09 May 2019 |
| | | US | 2019-0230070 | A1 | 25 July 2019 |
| | | US | 2019-0281030 | A1 | 12 September 2019 |

Form PCT/ISA/210 (patent family annex) (July 2022)

**INTERNATIONAL SEARCH REPORT**
Information on patent family members

| International application No. |
|---|
| **PCT/KR2023/004638** |

| Patent document cited in search report | | | Publication date (day/month/year) | Patent family member(s) | | | Publication date (day/month/year) |
|---|---|---|---|---|---|---|---|
| | | | | US | 2019-0306137 | A1 | 03 October 2019 |
| | | | | US | 2019-0356641 | A1 | 21 November 2019 |
| | | | | US | 2020-0014670 | A1 | 09 January 2020 |
| | | | | US | 2020-0014671 | A1 | 09 January 2020 |
| | | | | US | 2020-0069734 | A1 | 05 March 2020 |
| | | | | US | 2020-0076782 | A1 | 05 March 2020 |
| | | | | US | 2020-0127985 | A1 | 23 April 2020 |
| | | | | US | 2020-0178669 | A1 | 11 June 2020 |
| | | | | US | 2020-0195623 | A1 | 18 June 2020 |
| | | | | US | 2020-0242682 | A1 | 30 July 2020 |
| | | | | US | 2020-0358749 | A1 | 12 November 2020 |
| | | | | US | 2020-0382480 | A1 | 03 December 2020 |
| | | | | US | 2020-0402138 | A1 | 24 December 2020 |
| | | | | US | 2021-0024959 | A1 | 28 January 2021 |
| | | | | US | 2021-0087537 | A1 | 25 March 2021 |
| | | | | US | 2021-0163895 | A1 | 03 June 2021 |
| | | | | US | 2021-0174426 | A1 | 10 June 2021 |
| | | | | US | 2021-0174427 | A1 | 10 June 2021 |
| | | | | US | 2021-0174428 | A1 | 10 June 2021 |
| | | | | US | 2021-0174429 | A1 | 10 June 2021 |
| | | | | US | 2021-0180017 | A1 | 17 June 2021 |
| | | | | US | 2021-0326964 | A1 | 21 October 2021 |
| | | | | US | 2021-0350443 | A1 | 11 November 2021 |
| | | | | US | 2021-0358015 | A1 | 18 November 2021 |
| | | | | US | 2022-0148073 | A1 | 12 May 2022 |
| | | | | US | 2022-0156820 | A1 | 19 May 2022 |
| | | | | US | 2023-0109515 | A1 | 06 April 2023 |
| | | | | US | 9292871 | B2 | 22 March 2016 |
| | | | | US | 9361638 | B2 | 07 June 2016 |
| | | | | US | 9373138 | B2 | 21 June 2016 |
| | | | | US | 9396491 | B2 | 19 July 2016 |
| | | | | US | 9430790 | B2 | 30 August 2016 |
| | | | | US | 9430794 | B2 | 30 August 2016 |
| | | | | US | 9436957 | B2 | 06 September 2016 |
| | | | | US | 9449338 | B2 | 20 September 2016 |
| | | | | US | 9466081 | B2 | 11 October 2016 |
| | | | | US | 9524519 | B2 | 20 December 2016 |
| | | | | US | 9734526 | B2 | 15 August 2017 |
| | | | | US | 9767520 | B2 | 19 September 2017 |
| | | | | US | 9824408 | B2 | 21 November 2017 |
| | | | | US | 9922380 | B2 | 20 March 2018 |
| | | | | US | 9922381 | B2 | 20 March 2018 |
| | | | | WO | 2015-153474 | A1 | 08 October 2015 |
| | | | | WO | 2018-204822 | A1 | 08 November 2018 |
| | | | | WO | 2019-126748 | A1 | 27 June 2019 |
| | | | | WO | 2019-191495 | A1 | 03 October 2019 |
| | | | | WO | 2020-046906 | A1 | 05 March 2020 |
| EP | 1734119 | A2 | 20 December 2006 | EP | 0954576 | A2 | 10 November 1999 |
| | | | | EP | 1734119 | A3 | 05 December 2012 |
| | | | | JP | 11-511649 | A | 12 October 1999 |

Form PCT/ISA/210 (patent family annex) (July 2022)

**INTERNATIONAL SEARCH REPORT**
Information on patent family members

International application No.

**PCT/KR2023/004638**

| Patent document cited in search report | Publication date (day/month/year) | Patent family member(s) | | | Publication date (day/month/year) |
|---|---|---|---|---|---|
| | | KR | 10-0464923 | B1 | 13 June 2005 |
| | | KR | 10-1999-0008285 | A | 25 January 1999 |
| | | US | 5998166 | A | 07 December 1999 |
| | | US | 6444789 | B1 | 03 September 2002 |
| | | WO | 96-34953 | A2 | 07 November 1996 |
| | | WO | 96-34953 | A3 | 05 December 1996 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20210110827

**Non-patent literature cited in the description**

- **SCHEIT**. Nucleotide Analogs. John Wiley, 1980
- **UHLMAN** ; **PEYMAN**. *Chemical Reviews*, 1990, vol. 90, 543-584